# EUROPEAN PATENT SPECIFICATION

(11) **EP 0 656 063 B1**
(45) Date of publication and mention of the grant of the patent: **01.12.1999**
(21) Application number: 93919553.3
(22) Date of filing: 10.08.1993
(51) Int. Cl.: C12N 15/30, C07K 14/44, C07K 16/20, G01N 33/569, C12Q 1/68

(54) **NUCLEOTIDE SEQUENCES CODING FOR CRYPTOSPORIDIUM PROTEINS, POLYPEPTIDES CODED BY SAID SEQUENCES AND KITS FOR THE USE THEREOF**
NUKLEOTIDSEQUENZEN DIE FUR PROTEINE VON CRYPTOSPORIDIUM KODIEREN, POLYPEPTIDE, WELCHE DURCH DIESE SEQUENZEN KODIERT WERDEN, UND KITS FUR DEREN BENUTZUNG
SEQUENCES NUCLEOTIDIQUES CODANT LES PROTEINES DE CRYPTOSPORIDIUS, POLYPEPTIDES CODES PAR LESDITES SEQUENCES ET LEURS MATERIELS D'UTILISATION

(30) Priority: 13.08.1992 IT RM920603
(43) Date of publication of application: 07.06.1995
(73) Proprietor: UNIVERSITA' DEGLI STUDI DI ROMA "LA SAPIENZA", 00185 Roma (IT)
(72) Inventor: LA ROSA, Giuseppe, Istituto Superiore di Sanità, I-00161 Roma (IT); GOMEZ MORALES, Maria Angeles, I-00161 Roma (IT); POZIO, Edoardo, I-00161 Roma (IT); CRISANTI, Andrea, Univ. degli Studi di Roma, I-00185 Roma (IT); MULLER, Hans Michael, Univ. degli Studi di Roma, I-00185 Roma (IT); RANUCCI, Lorella, Univ. degli Studi di Roma, I-00185 Roma (IT); RECKMANN, Ingeborg, Univ. degli Studi di Roma, I-00185 Roma (IT); COLUZZI, Mario, Univ. degli Studi di Roma, I-00185 Roma (IT)
(74) Representative: de Simone, Domenico
(86) International application number: IT9300089
(87) International publication number: WO9404681

(56) References cited:
- INFECTION AND IMMUNITY vol. 61, no. 6 , June 1993 , WASHINGTON US pages 2347 - 2356 RANUCCI, L. ET AL. 'Characterization and immunolocalization of a Cryptosporidium protein containing repeated amino acid motifs'
- MOLECULAR AND BIOCHEMICAL PARASITOLOGY vol. 56, no. 1 , November 1992 pages 69 - 78 LALLY, N. C. ET AL. 'A 2359-base pair DNA fragment from Cryptosporidium parvum encoding a repetitive oocyst protein'
- J. PROTOZOOL. vol. 38, no. 6 , November 1991 pages 76S - 78S DYKSTRA, C. C. ET AL. 'Construction of genomic libraries of Cryptosporidium parvum and identification of antigen-encoding genes'

### SEQUENCE LISTING

(1) GENERAL INFORMATION:
   (i) APPLICANT:
      (A) NAME: Universita' degli Studi di Roma "La Sapienza"
      (B) STREET: P.le Aldo Moro 5
      (C) CITY: Rome
      (E) COUNTRY: Italy
      (F) POSTAL CODE (ZIP): 00185

      (A) NAME: Istituto Superiore di Sanita'
      (B) STREET: V.le Regina Elena 299
      (C) CITY: Rome
      (E) COUNTRY: Italy
      (F) POSTAL CODE (ZIP): 00161
   (ii) TITLE OF INVENTION: Nucleotide sequences coding for Cryptosporidium proteins, polypeptides coded by said sequences and kits
   (iii) NUMBER OF SEQUENCES: 2
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.25 (EPO)
(2) INFORMATION FOR SEQ ID NO: 1:
   (i) SEQUENCE CHARACTERISTICS:
      (A) LENGTH: 2359 base pairs
      (B) TYPE: nucleic acid
      (C) STRANDEDNESS: double
      (D) TOPOLOGY: linear
   (ii) MOLECULE TYPE: DNA (genomic)
   (iii) HYPOTHETICAL: NO
   (iii) ANTI-SENSE: NO
   (vi) ORIGINAL SOURCE:
      (A) ORGANISM: Cryptosporidium parvum
   (ix) FEATURE:
      (A) NAME/KEY: CDS
      (B) LOCATION: 1..2359
      (xi) SEQUENCE DESCRIPTION: SEQ ID NO: 1:
   GAA TTC GAA TGC CCA CCA GGT ACA ATT TTA AAA GAT GAT CAA TGT CAA    48
   Glu Phe Glu Cys Pro Pro Gly Thr Ile Leu Lys Asp Asp Gln Cys Gln
    1               5                  10                  15
   TCG ATA GAA AGA GTT GAT ACA ATT TGT CCA CCA GGG TTT GTA GAT AAT    96
   Ser Ile Glu Arg Val Asp Thr Ile Cys Pro Pro Gly Phe Val Asp Asn
               20                  25                  30
   GGC GAA GAT TGT GTC CAA TTT TCT GCA CCA GAG AAA ATT TGC CCC CAA   144
   Gly Glu Asp Cys Val Gln Phe Ser Ala Pro Glu Lys Ile Cys Pro Gln
           35                  40                  45
   GGA TTT TCT CTT TCC GGA AAA CAA TGT GTT AAA ACA GAA TCT GCT CCA   192
   Gly Phe Ser Leu Ser Gly Lys Gln Cys Val Lys Thr Glu Ser Ala Pro
       50                  55                  60
   AGA TTA ACA GAA TGC CCA CCA GGT ACA ACC TTG GAA AAT AAC AGT TGT   240
   Arg Leu Thr Glu Cys Pro Pro Gly Thr Thr Leu Glu Asn Asn Ser Cys
    65                  70                  75                  80
   ATT TCA TAT GAA CTA GAA GAT GCC ATT TGT CCA CCT GGA TAT CTC GAC   288
   Ile Ser Tyr Glu Leu Glu Asp Ala Ile Cys Pro Pro Gly Tyr Leu Asp
                   85                  90                  95
   AAT GGA TCA GAC TGT GTT CAG TTT TCT CAA CCA GAA AAG GAG TGT CCA   336
   Asn Gly Ser Asp Cys Val Gln Phe Ser Gln Pro Glu Lys Glu Cys Pro
               100                 105                 110
   ACA GGT TTT GTA TTA ATT GGA AAA CAA TGT ACC CAA ACT ACT CAA GCT   384
   Thr Gly Phe Val Leu Ile Gly Lys Gln Cys Thr Gln Thr Thr Gln Ala
           115                 120                 125
   CCA CCA CAA CCA GAG TGT CCT CCA GGT ACA AAC CTG GTA AAT GGA CAA   432
   Pro Pro Gln Pro Glu Cys Pro Pro Gly Thr Asn Leu Val Asn Gly Gln
       130                 135                 140
   TGC CAA AAA GTT GAA AGG ATA AAT ATG GTA TGT CCA ACT GGT TTT ATT   480
   Cys Gln Lys Val Glu Arg Ile Asn Met Val Cys Pro Thr Gly Phe Ile
   145                 150                 155                 160
   GAT AAT GGT ACA AAT TGT GCT TCT TTC TCC GCA CCA AAC AGA GAA TGC   528
   Asp Asn Gly Thr Asn Cys Ala Ser Phe Ser Ala Pro Asn Arg Glu Cys
                   165                 170                 175
   CCA CCT GGA TAT ACA CTT TCT GGA TCC CAA TGC GAG CAA ATA AAA GAA   576
   Pro Pro Gly Tyr Thr Leu Ser Gly Ser Gln Cys Glu Gln Ile Lys Glu
               180                 185                 190
   GCA CCT CCT GTT TCA GAA TGT CCA CCA GGA TAT AAA CTT CAA GGA AAT   624
   Ala Pro Pro Val Ser Glu Cys Pro Pro Gly Tyr Lys Leu Gln Gly Asn
           195                 200                 205
   CAA TGT ACT GCA CTA AAA ATG ATC GAT GCT ATC TGC CCA GAT GGA TTT   672
   Gln Cys Thr Ala Leu Lys Met Ile Asp Ala Ile Cys Pro Asp Gly Phe
       210                 215                 220
   TTA CCA AAT GGA GAC GAT TGT ATC CAA TTT TCT CCT GCT TCA ACT GTA   720
   Leu Pro Asn Gly Asp Asp Cys Ile Gln Phe Ser Pro Ala Ser Thr Val
   225                 230                 235                 240
   TGT CCT ACT GGA TTC ACT CTA CAA AAT CAA CAG TGT GTT CAA ACA ACT   768
   Cys Pro Thr Gly Phe Thr Leu Gln Asn Gln Gln Cys Val Gln Thr Thr
                   245                 250                 255
   ACC TCA CCA AAA ACA CCA GAA TGT CCT CCA GGT TCT GCG TTG GAT GGA   816
   Thr Ser Pro Lys Thr Pro Glu Cys Pro Pro Gly Ser Ala Leu Asp Gly
               260                 265                 270
   GAC TCG TGC ACA AGA CTT GTT CCC GGG GCT CTT CAA TAC GTT TGT CCT   864
   Asp Ser Cys Thr Arg Leu Val Pro Gly Ala Leu Gln Tyr Val Cys Pro
           275                 280                 285
   GTT GGT ACT AGA GAG GGG GAC GTT TGC GTA GAG AGA TCG ATT AGT TCG   912
   Val Gly Thr Arg Glu Gly Asp Val Cys Val Glu Arg Ser Ile Ser Ser
       290                 295                 300
   CCT GTT TTG GAA TGC CCA CCT GGT TAT TCA TTG GAA ACA GGT AAA CAA   960
   Pro Val Leu Glu Cys Pro Pro Gly Tyr Ser Leu Glu Thr Gly Lys Gln
   305                 310                 315                 320
   TGT GTT AGA AGA AGC CAA TAT GAC TGT TCA GTA ACA ACT TAT GTT ACA 1008
   Cys Val Arg Arg Ser Gln Tyr Asp Cys Ser Val Thr Thr Tyr Val Thr
                   325                 330                 335
   GAG TGT AAA ACA CCT GAT GTT AAA GCA CTA AGA AGA TTA GCA GCT GCA 1056
   Glu Cys Lys Thr Pro Asp Val Lys Ala Leu Arg Arg Leu Ala Ala Ala
               340                 345                 350
   AAA GAA ACA TCA ACA GTT TAT GAA ACA TCT GAG ATA CAA AAT CCA GGA 1104
   Lys Glu Thr Ser Thr Val Tyr Glu Thr Ser Glu Ile Gln Asn Pro Gly
           355                 360                 365
   CAT CAT CAT GGT CAT TCT CAT GGG CAT TCA CAT TCA CAA GTT ATA CCA 1152
   His His His Gly His Ser His Gly His Ser His Ser Gln Val Ile Pro
       370                 375                 380
   ATT CAA ACC CAG AAT ATA CAT ACA CAA CAT CAT AAA GAG GCT CCA AGG 1200
   Ile Gln Thr Gln Asn Ile His Thr Gln His His Lys Glu Ala Pro Arg
   385                 390                 395                 400
   CCA ATT TGT GAA GAT GTT CCA AAA ATT ACC CCA AAA ACT TGT ACA AAA 1248
   Pro Ile Cys Glu Asp Val Pro Lys Ile Thr Pro Lys Thr Cys Thr Lys
                   405                     410                 415
   GCT GAT TCT GTC CCA GCT GTG CCT ATT TGC GAG AAC AAT GCT GAA CTT 1296
   Ala Asp Ser Val Pro Ala Val Pro Ile Cys Glu Asn Asn Ala Glu Leu
               420                 425                     430
   GTA GGA AAA GAA TGT GTA TTA ACA AAT TAC TAC CCA TTA GAA GCA ATT 1344
   Val Gly Lys Glu Cys Val Leu Thr Asn Tyr Tyr Pro Leu Glu Ala Ile
           435                 440                 445
   TGT CAA GAT GGA ACA AGA TCA AAA GAG TGT GCT AAG TTT GTA AAA ACT 1392
   Cys Gln Asp Gly Thr Arg Ser Lys Glu Cys Ala Lys Phe Val Lys Thr
       450                 455                 460
   CCA CCT ACT TTA AAA TGT CCG CCA GGT TCT GTA GAT GTA GGA TCT CAA 1440
   Pro Pro Thr Leu Lys Cys Pro Pro Gly Ser Val Asp Val Gly Ser Gln
   465                 470                 475                 480
   TGT CAA GTT AAC AAA TAT TCA CCA TAT GAT CTT GCA TGC CCT GCA GGA 1488
   Cys Gln Val Asn Lys Tyr Ser Pro Tyr Asp Leu Ala Cys Pro Ala Gly
                   485                 490                 495
   TAT GCA TTG GTT GGA GAC AAA TGC GCT ACC ACA AGA GAA AAA GTT TGC 1536
   Tyr Ala Leu Val Gly Asp Lys Cys Ala Thr Thr Arg Glu Lys Val Cys
               500                 505                 510
   CCG AAT GAA AGT TGC CAA AGA GTT GTA ACT GCG CCT GTT TCT TTA ACT 1584
   Pro Asn Glu Ser Cys Gln Arg Val Val Thr Ala Pro Val Ser Leu Thr
           515                 520                 525
   TGT CCC CCT GGA TAT CAC CAA ATA GAT GAA GTT ATG AAT ATT TCT GCT 1632
   Cys Pro Pro Gly Tyr His Gln Ile Asp Glu Val Met Asn Ile Ser Ala
       530                 535                 540
   CAT CCA CAC CAC AGA CAC TTA GCT GGG GTT CAA TCT ACT TCT CAA AAG 1680
   His Pro His His Arg His Leu Ala Gly Val Gln Ser Thr Ser Gln Lys
   545                 550                 555                 560
   GGA TAT TCT CAT GGA CAT AAA TAT ACT CCT GTA ATT TCT CAG CCA CCA 1728
   Gly Tyr Ser His Gly His Lys Tyr Thr Pro Val Ile Ser Gln Pro Pro
                   565                 570                 575
   CAA CCA GTT CCA GTT GTT GCT CCT ATT CAG CAA ATG AAA TGC ATC CAT 1776
   Gln Pro Val Pro Val Val Ala Pro Ile Gln Gln Met Lys Cys Ile His
               580                 585                 590
   GCA AAC CAT GCT CCA TAT AAT CTT ATC TGT CCT GTT GGA TCA AGA CTT 1824
   Ala Asn His Ala Pro Tyr Asn Leu Ile Cys Pro Val Gly Ser Arg Leu
           595                 600                 605
   GTA GCG GAT AAA TGT GTT ACA TAT TCG GAT AAA ATA TGT CCA AAT GGT 1872
   Val Ala Asp Lys Cys Val Thr Tyr Ser Asp Lys Ile Cys Pro Asn Gly
       610                 615                 620
   AAT TGC GAG CGT ATA TAT AAT GAG CCT GCT GAA TTA GTA TGC CCT CCA 1920
   Asn Cys Glu Arg Ile Tyr Asn Glu Pro Ala Glu Leu Val Cys Pro Pro
   625                 630                 635                 640
   GGA TTC TCA TCA TCT AAA CCA ATT CAG CCA ATA AGC CAT TCT CAT ATT 1968
   Gly Phe Ser Ser Ser Lys Pro Ile Gln Pro Ile Ser His Ser His Ile
                   645                 650                 655
   AAC CAT CCA AAT GTT TCT GTT CCC GTC CAA CCA CAA ACT ATT AAC CAA 2016
   Asn His Pro Asn Val Ser Val Pro Val Gln Pro Gln Thr Ile Asn Gln
               660                 665                 670
   CCA CAA GTA ATT CAA CAA AGA CAA GTA AAT TAT CAG CCA CAA GTA ATT 2064
   Pro Gln Val Ile Gln Gln Arg Gln Val Asn Tyr Gln Pro Gln Val Ile
           675                 680                 685
   CAT CAA ACA CAG GAA ATT TTA ACA ACT TAT CCA ACT CCA GTT TAC CAA 2112
   His Gln Thr Gln Glu Ile Leu Thr Thr Tyr Pro Thr Pro Val Tyr Gln
       690                 695                 700
   ACC GGC ACA ATT TAT CAA GGA CAT CAT CAT CAT CAT CAT CAT CAT CAC 2160
   Thr Gly Thr Ile Tyr Gln Gly His His His His His His His His His
   705                 710                 715                 720
   AGA AAT CTA GCT TCC CCT GAG TGC ATT AAG ACA ATT TCA GTA CCT TAT 2208
   Arg Asn Leu Ala Ser Pro Glu Cys Ile Lys Thr Ile Ser Val Pro Tyr
                   725                 730                 735
   ATT TTA AAA TGC GAA TCT CCA TTT ATT TTA GAT GGC GAC AAA TGT ATC 2256
   Ile Leu Lys Cys Glu Ser Pro Phe Ile Leu Asp Gly Asp Lys Cys Ile
               740                 745                 750
   GAA AAA ACA GAA AAA ATT TGT CTA CAA GGT GAC TGC AGA AAA CAA GTC 2304
   Glu Lys Thr Glu Lys Ile Cys Leu Gln Gly Asp Cys Arg Lys Gln Val
           755                 760                 765
   GTC GTT CCA CCA ACT CTT TCA TGT CCA CAA GGT TAC AGA AAT GCC AAC 2352
   Val Val Pro Pro Thr Leu Ser Cys Pro Gln Gly Tyr Arg Asn Ala Asn
       770                 775                 780
   GGA ATT C                                                       2359
   Gly Ile
   785
(2) INFORMATION FOR SEQ ID NO: 2:
   (i) SEQUENCE CHARACTERISTICS:
      (A) LENGTH: 786 amino acids
      (B) TYPE: amino acid
      (D) TOPOLOGY: linear
   (ii) MOLECULE TYPE: protein
   (xi) SEQUENCE DESCRIPTION: SEQ ID NO: 2:
   Glu Phe Glu Cys Pro Pro Gly Thr Ile Leu Lys Asp Asp Gln Cys Gln
    1              5                   10                  15
   Ser Ile Glu Arg Val Asp Thr Ile Cys Pro Pro Gly Phe Val Asp Asn
               20                  25                  30
   Gly Glu Asp Cys Val Gln Phe Ser Ala Pro Glu Lys Ile Cys Pro Gln
           35                  40                  45
   Gly Phe Ser Leu Ser Gly Lys Gln Cys Val Lys Thr Glu Ser Ala Pro
       50                  55                  60
   Arg Leu Thr Glu Cys Pro Pro Gly Thr Thr Leu Glu Asn Asn Ser Cys
   65                  70                  75                  80
   Ile Ser Tyr Glu Leu Glu Asp Ala Ile Cys Pro Pro Gly Tyr Leu Asp
                   85                  90                  95
   Asn Gly Ser Asp Cys Val Gln Phe Ser Gln Pro Glu Lys Glu Cys Pro
               100                 105                 110
   Thr Gly Phe Val Leu Ile Gly Lys Gln Cys Thr Gln Thr Thr Gln Ala
           115                 120                 125
   Pro Pro Gln Pro Glu Cys Pro Pro Gly Thr Asn Leu Val Asn Gly Gln
       130                 135                 140
   Cys Gln Lys Val Glu Arg Ile Asn Met Val Cys Pro Thr Gly Phe Ile
   145                 150                 155                 160
   Asp Asn Gly Thr Asn Cys Ala Ser Phe Ser Ala Pro Asn Arg Glu Cys
                   165                 170                 175
   Pro Pro Gly Tyr Thr Leu Ser Gly Ser Gln Cys Glu Gln Ile Lys Glu
               180                 185                 190
   Ala Pro Pro Val Ser Glu Cys Pro Pro Gly Tyr Lys Leu Gln Gly Asn
           195                 200                 205
   Gln Cys Thr Ala Leu Lys Met Ile Asp Ala Ile Cys Pro Asp Gly Phe
       210                 215                 220
   Leu Pro Asn Gly Asp Asp Cys Ile Gln Phe Ser Pro Ala Ser Thr Val
   225                 230                 235                 240
   Cys Pro Thr Gly Phe Thr Leu Gln Asn Gln Gln Cys Val Gln Thr Thr
                   245                 250                 255
   Thr Ser Pro Lys Thr Pro Glu Cys Pro Pro Gly Ser Ala Leu Asp Gly
               260                 265                 270
   Asp Ser Cys Thr Arg Leu Val Pro Gly Ala Leu Gln Tyr Val Cys Pro
           275                 280                 285
   Val Gly Thr Arg Glu Gly Asp Val Cys Val Glu Arg Ser Ile Ser Ser
       290                 295                 300
   Pro Val Leu Glu Cys Pro Pro Gly Tyr Ser Leu Glu Thr Gly Lys Gln
   305                 310                 315                 320
   Cys Val Arg Arg Ser Gln Tyr Asp Cys Ser Val Thr Thr Tyr Val Thr
                   325                 330                 335
   Glu Cys Lys Thr Pro Asp Val Lys Ala Leu Arg Arg Leu Ala Ala Ala
               340                 345                 350
   Lys Glu Thr Ser Thr Val Tyr Glu Thr Ser Glu Ile Gln Asn Pro Gly
           355                 360                 365
   His His His Gly His Ser His Gly His Ser His Ser Gln Val Ile Pro
       370                 375                 380
   Ile Gln Thr Gln Asn Ile His Thr Gln His His Lys Glu Ala Pro Arg
   385                 390                 395                 400
   Pro Ile Cys Glu Asp Val Pro Lys Ile Thr Pro Lys Thr Cys Thr Lys
                   405                 410                 415
   Ala Asp Ser Val Pro Ala Val Pro Ile Cys Glu Asn Asn Ala Glu Leu
               420                 425                 430
   Val Gly Lys Glu Cys Val Leu Thr Asn Tyr Tyr Pro Leu Glu Ala Ile
           435                 440                 445
   Cys Gln Asp Gly Thr Arg Ser Lys Glu Cys Ala Lys Phe Val Lys Thr
       450                 455                 460
   Pro Pro Thr Leu Lys Cys Pro Pro Gly Ser Val Asp Val Gly Ser Gln
   465                 470                 475                 480
   Cys Gln Val Asn Lys Tyr Ser Pro Tyr Asp Leu Ala Cys Pro Ala Gly
                   485                 490                 495
   Tyr Ala Leu Val Gly Asp Lys Cys Ala Thr Thr Arg Glu Lys Val Cys
               500                 505                 510
   Pro Asn Glu Ser Cys Gln Arg Val Val Thr Ala Pro Val Ser Leu Thr
           515                 520                 525
   Cys Pro Pro Gly Tyr His Gln Ile Asp Glu Val Met Asn Ile Ser Ala
       530                 535                 540
   His Pro His His Arg His Leu Ala Gly Val Gln Ser Thr Ser Gln Lys
   545                 550                 555                 560
   Gly Tyr Ser His Gly His Lys Tyr Thr Pro Val Ile Ser Gln Pro Pro
                   565                 570                 575
   Gln Pro Val Pro Val Val Ala Pro Ile Gln Gln Met Lys Cys Ile His
               580                 585                 590
   Ala Asn His Ala Pro Tyr Asn Leu Ile Cys Pro Val Gly Ser Arg Leu
           595                 600                 605
   Val Ala Asp Lys Cys Val Thr Tyr Ser Asp Lys Ile Cys Pro Asn Gly
       610                 615                 620
   Asn Cys Glu Arg Ile Tyr Asn Glu Pro Ala Glu Leu Val Cys Pro Pro
   625                 630                 635                 640
   Gly Phe Ser Ser Ser Lys Pro Ile Gln Pro Ile Ser His Ser His Ile
                   645                 650                 655
   Asn His Pro Asn Val Ser Val Pro Val Gln Pro Gln Thr Ile Asn Gln
               660                 665                 670
   Pro Gln Val Ile Gln Gln Arg Gln Val Asn Tyr Gln Pro Gln Val Ile
           675                 680                 685
   His Gln Thr Gln Glu Ile Leu Thr Thr Tyr Pro Thr Pro Val Tyr Gln
       690                 695                 700
   Thr Gly Thr Ile Tyr Gln Gly His His His His His His His His His
   705                 710                 715                 720
   Arg Asn Leu Ala Ser Pro Glu Cys Ile Lys Thr Ile Ser Val Pro Tyr
                   725                 730                 735
   Ile Leu Lys Cys Glu Ser Pro Phe Ile Leu Asp Gly Asp Lys Cys Ile
               740                 745                 750
   Glu Lys Thr Glu Lys Ile Cys Leu Gln Gly Asp Cys Arg Lys Gln Val
           755                 760                 765
   Val Val Pro Pro Thr Leu Ser Cys Pro Gln Gly Tyr Arg Asn Ala Asn
       770                 775                 780
   Gly Ile
   785

## Description

The invention concerns nucleic acids and polypeptides coded by said nucleic acids, derived from protozoan parasites of *Cryptosporidium* genus. Nucleic acids and peptides are advantageously used for developing detection assays of *Cryptosporidium* in biological samples of human and animal origin and/or in the environment.

*Cryptosporidium* parasites infect the intestinal tract of several animal species. Over the last decade the number of infections in humans has dramatically increased. Most of the affected patients show a marked immunodeficiency, with a high incidence of AIDS. The immunocompromised patients develop a severe and frequently irreversible diarrhoea which causes malnutrition and represents a major factor leading to death.

The prophylaxis of *Cryptospcridium* infections is hampered by the lack of reliable immunoassays for the detection of the parasite. Moreover, by microscopic examination, parasite oocyst are difficult to discriminate from several microoganisms that are morphologically similar to *Cryptosporidium,* like *Candida* species.

The development of immunological and molecular diagnostic assays highly specific for *Cryptosporidium* absolutely requires the biochemical characterisation and localization of parasite antigens and the cloning of corresponding genes. The availability of diagnostic assays would permit to develop prophylactic measures for immunodeficient patients by detecting the parasites in the environment (food, water) and in individuals (relatives, nurses) that may act as carriers.

Moreover, no effective therapeutic compounds against *Cryptosporidium* infection are available; therefore there is the need to develop reagents able to prevent the onset of the infection in immunodeficient patients.

The authors of the present invention have obtained a rabbit antiserum raised against a whole lysate of *Cryptosporidium* oocysts. The serum has been used to screen a genomic library of *Cryptosporidium* from infected intestinal mucosal cells, into the expression vector λgt11. Among clones isolated, clone cpRL3 has been shown to comprise a 2359 bp (SEQ ID No.1) insert with an open reading frame coding for a polypeptide of 786 amino acids (SEQ ID No.2). Scanning of the 67.0 version of the "GENE BANK" data base using the cpRL3 sequence failed to reveal any similarity with known DNA sequences.

The cpRL3 insert has been subcloned into an expression plasmid and the corresponding recombinant polypeptide is produced in *E. coli,* fused at the N-terminus to a stretch of six histidines. The histidine sequence allows a fast and efficient purification by nickel chelate chromatography of the polypeptide encoded by cpRL3. The recombinant polypeptide is used as immunogen in Balb/c mice for producing antisera and monoclonal antibodies. The recombinant protein is highly immunogenic. This sequence codes for a portion of a protein of the oocyst wall of Cryptosporidium that has an apparent molecular weight of 190,000 Dalton, named "Cryptosporidium Oocyst Wall Protein" (COWP).

Nucleotide and polypeptide sequences derived from the isolated sequence are advantageously utilized for diagnosis of *Cryptosporidium* infections in patients and/or animals and for analysis of environmental contamination by *Cryptosporidium* oocysts. Treatment of *Cryptosporidium* infection may be obtained by administration of antibodies raised against COWP.

The invention concerns also: - designing of specific oligonucleotides, as primers in polymerase chain reactions (PCR) reactions for the detection of the COWP DNA sequence; - the utilisation of COWP amino acid sequence or fragments thereof to raise antisera and/or monoclonal antibodies for the development of immunological assays to detect the presence of *Cryptosporidium* and/or COWP molecule; - the use of COWP derived polypeptides, either synthetic or recombinant, as components of diagnostic kits for the detection of COWP released by parasites; - the utilisation of COWP-derived polypeptides, either synthetic or recombinant, for producing antisera and/or monoclonal antibodies to be employed in the therapy of *Cryptosporidiosis.*

It is a specific object of the present invention a polypeptide of the oocyst wall of *Cryptosporidium* having a molecular weight of 190.000 D as determined by SDS polyacrilamide gel electrophoresis and comprising a contiguous sequence encoded by a nucleotide sequence of SEQ ID No. 1, or parts or variants thereof reacting with antibodies developed against the expression product of the nucleotide sequence of SEQ ID No. 1. Preferably said polypeptide consists of a contiguous sequence encoded by the nucleotide sequence of SEQ ID No. 1 or by parts or variants thereof reacting with antibodies developed against the expression product of the nucleotide sequence of SEQ ID No. 1; most preferably said contiguous sequence is comprised in the aminoacid sequence of SEQ ID No. 2.

It is another object of the invention a diagnostic kit for the detection of *Cryptosporidium* in biological and environmental samples comprising, as specific ligand, the polypeptide according to the invention.

It is another object of the invention the use of a polypeptide according to the invention for raising antibodies able to detect *Cryptosporidium* infection in biological and environmental samples.

It is another object of the invention an antibody obtained using as immunogen a polypeptide according to the invention.

It is another object of the invention the use of the antibody for the specific detection of *Cryptosporidium* or of *Cryptosporidium* oocyst wall protein.

It is another object of the invention a diagnostic kit for the detection of *Cryptosporidium* in biological and environmental samples comprising, as specific ligand, an antibody able to react with at least one polypeptide according to the invention.

It is another object of the invention an oligonucleotide having a sequence comprised in the nucleotide sequence of SEQ ID No. 1, or in the complementary strand of SEQ ID No. 1 able to hybridize selectively to nucleotide sequences of *Cryptosporidium.*

It is another object of the invention a diagnostic kit for the detection of *Cryptosporidium* in biological and environmental samples comprising, as specific ligand, the oligonucleotide according to the invention.

It is another object of the invention a PCR kit for the amplification of *Cryptosporidium* DNA comprising, as specific primer, at least one oligonucleotide according to the invention. Preferably said PCR kit comprises two oligonucleotides having nucleotide sequences according to the invention. More preferably the two oligonucleotides have the sequence from nt. 722 to nt. 742 of SEQ ID No. 1 and from nt. 1828 to nt. 1852 of the complementary strand of SEQ ID No. 1, respectively.

The invention will be illustrated in the following examples, by making reference to the following figures, wherein:

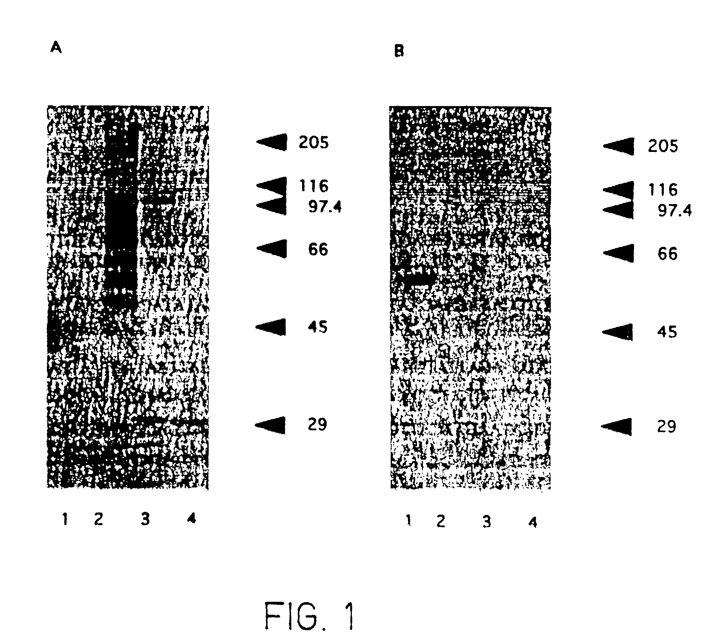

- Figure 1 shows an immunoblot analysis of the mouse serum (M10/01 (A) and of a control mouse serum anti TRAP (B) against: the expression product of the control plasmid pDS56/RBSII-E-6his-TRAP purified on nickel column (1); the expression product of the plasmid pDS56/RBSII-E-6his-cpRL3 purified on nickel column (2); a protein lysate of *E. coli* cells transformed with pDS56/RBSII-E-6his-cpRL3 induced with IPTG (3); and non induced (4); molecular weight standard are indicated.

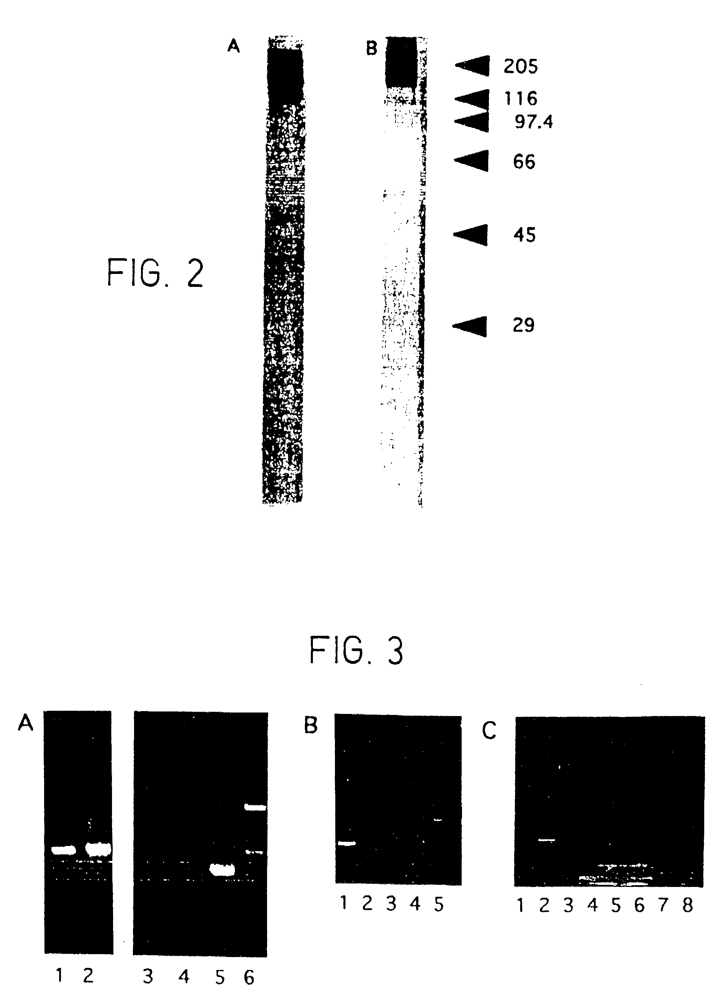

- Figure 2 shows an immunoblot analysis using the mouse serumM10/01 (A) and the MAbl IB2 (B) against a protein lysate of *C. parvum* oocysts.
- Figure 3A shows an electrophoresis of PCR products amplified from C. parvum DNA (1; 4) and from DNA of the plasmid pDS56/RBSII-E-6his-cpRL3 (2;5) using primers combinations Cry-3/Cry-6 (1;2) or Cry-5/Cry-6 (3;4;5;). As control, PCR reaction without template DNA (3). Figure 3B shows an electrophoresis of PCR products amplified from DNA of several parasite species using primer combination Cry-3/Cry-6: DNA extracted from: *C. parvum* (1), *Sarcocystis sp.* (2), *Giardia lamblie* (3) and *P. falciparum* (4). Figure 3C shows an electrophoresis of PCR products amplified from DNA of progressively diluted *C. parvum* oocysts using the primer combinations Cry-3/Cry-6: molecular standards (1), 160 oocysts (2), 80 oocysts (3), 40 oocysts (4), 20 oocysts (5) 10 oocysts (6). As control, PCR reaction is done without template DNA (7) or in the presence of *P. falciparum* DNA (8). In panels A, B and C, lanes 6, 5 and 1 respectively, DNA markers are 3611; 1166; 606; 517; 396; 318; 263 bp.

### Example 1 λgt11 library with DNA extracted from C. parvum infected calf intestinal mucosa

To develop a *C. parvum* genomic expression library, DNA extracted from the intestinal mucosa cf an infected calf is used.

A newborne calf is infected with 6 x 10⁸ oocysts of *C. parvum* MI ISS-1 (Pozio et al. 1992. Trans. R. Soc. Trop. Med. Hyg. 86:636-638). After 5 days, the gut is opened, cut into segments of 30 cm each and washed in phosphate-buffered saline (PBS). Nitro-cellulose filters (soaked in PBS) of the same size as the gut segments are applied to the mucosal side for a few seconds. Filters are progressively numbered and processed for DNA extraction. A small sample is removed from each filter and analysed by microscopy to determine whether parasites have been removed. The sample from each filter is incubated with 1% glutaraldehyde in cacodylate buffer for 2 h. The filters are dehydrated through an increasing ethanol series, embedded in Epon, and cured at 60°C for 24 h. Sections are cut at 0.2-µm thickness and stained with toluidine blue. The analysis reveals that nitro-cellulose filters remove only the superficial layer of mucosal cells, together with a large number of parasites.

Only DNA extracted from filters that have removed a large number of parasites are used. DNA is digested with EcoRI and cloned in λgt11 EcoRI digested, 3' end to the coding sequence of β-galactosidase gene. Phage DNA with cloned inserts is packaged *in vitro* (Boehringer Mannheim in *vitro* packaging kit) to generate the library. The quality of the library is evaluated by analysing the sizes of a subset of inserts by polymerase chain reaction (PCR) with oligonucleotides corresponding to the flanking sequences of the EcoRI site of the β-galactosidase gene. The library has a complexity of 4.5 X 10⁶ plaques and an estimated average insert size of 1,800 bp.

The expression library is analysed by use of a rabbit serum developed against purified oocysts of *C. parvum* MI ISS-1. The serum is used after removal of the background reactivity by several absorptions on filters soaked with bacterial and phage lysates. Specific antibodies bound to filters are detected by use of a second anti-rabbit antibody conjugated to alkaline phosphatase. An insert, named cpRL3, consisting of a 2,359-bp open reading frame encoding a polypeptide of 786 amino acids, is isolated. The lack of both a start codon and a stop codon indicates that the sequence represents part of the coding sequence of the isolated parasitic gene.

### Example 2 Expression of the cpRL3 sequence in E. coli

The DNA insert cpRL3 is cloned in the EcoRI site of plasmid pDS56/RBSII-E⁻ 6xHis (a pDS56/RBSII derived plasmid containing an EcoRI site in the polylinker). The expression unit of this vector is under the control of an isopropyl-β-thiogalactopyranoside (IPTG)-inducible promoter and yields a fusion between a stretch of 6 histidines and the amino terminus of the inserted sequences (Stuber et al. 1990, Eur. J. Immunol 20:819-824) . cpRL3 is expressed in *E. coli* M15 carrying the lac repressor-producing plasmid pUHA1. Induction is performed in LB medium for 4 h at 37°C; 1 mM IPTG is added when the cell density reaches an optical density at 600 nm of 0 6.

### Example 3 Purification of recombinant polypeptide 6x His-cpRL3

The expression product of the cpRL3 sequence (recombinant polypeptide 6xHis-cpRL3) is purified in a single-step procedure, by nickel chelate affinity chromatography (Stuber et al., ibid.). In brief, one litre of an induced culture of M15(pUHA1) cells carrying plasmid pDS56/RBSII-E-6xHis-cpRL3 is harvested and stirred for 3 h in 100 ml of 6 M guanidine hydrochloride, 100 mM Na₂HPO₄, pH 8. The suspension is centrifuged at 10,000 x g, and the supernatant is directly applied to a nickel column (NTA-resin, Diagen). After an equilibration step with 8 M urea, 100 mM NaH₂PO₄, 10 mM Tris, pH 8, 6xHis-cpRL3 is eluted by lowering the pH of the urea solution stepwise to pH 4. From one litre of culture, 1 mg of 6xHis-cpRL3 is obtained.

### Example 4 Immunoblotting

Parasite lysates are obtained from *Cryptosporidium* oocysts purified by Percoll gradient centrifugation (Waldman, E., et al. 1986. J. Clin. Microbiol. 23:199-200). Parasites are lysed by incubation of pellet of 2 x 10⁷ oocysts with 0.2 ml of sample buffer (33 mM Tris-HCl, pH 6.8, 190 mM glycerol, 0.1% SDS). Bacterial lysates are obtained by treatment of 10⁹ induced or non induced *E. coli* cells with 1 ml of sample buffer. Proteins in total cell lysates are separated by SDS-polyacrylamide gel electrophoresis (PAGE) (Laemmli, U.K. 1970. Nature (London) 227:680-685) and electroblotted onto nitro-cellulose filters (blotting buffer, 25 mM Tris, 192 mM glycine, 20% methanol). Non-specific adsorption of antibodies to the nitro-cellulose is prevented by saturation of the filters with 1% bovine serum albumin in 2x TBST (20 mM Tris-HCl, pH 8, 300 mM NaCl, 0.1% Tween 20) for 2 h at room temperature. Nitrocellulose filters are incubated with antibodies for 2 h at room temperature. After extensive washing with 2x TBST, antibodies bound to the filters are detected by use of goat anti-mouse immunoglobulin (heavy and light chains) conjugated to alkaline phosphates (Promega). Phosphatase activity is disclosed by incubation of the filters with 0.3 mg of Nitro Blue Tetrazolium and 0.15 mg of 5-bromo 4-chloro-3-indolyl phosphate per ml in 100 mM Tris-HCl (pH 9.5)-100 mM NaCl-5 mM MgCl₂.

### Example 5 Monoclonal antibody production

After purification by nickel chelate chromatography, recombinant polypeptide 6xHis-cpRL3 is used as immunogen to develop specific antisera and monoclonal antibodies. BALB/c mice are immunised three times with 50 µg of purified 6xHis-cpRL3 polypeptide in complete (for the first immunisation) or incomplete Freund's adjuvant. Five days after the last immunisation, mouse spleen cells are fused with x63 Ag 8653 myeloma cells and subsequently screened for antibody production (Kohler,G. and C. Milstein 1975 Nature (London) 256:495-497).

The supernatants of cultures from growing hybrids are tested in an enzyme-linked immunosorbent assay (ELISA) against 6xHisCpRL3. Immunised mice develop an antibody titer of 1/500,000. Figure 1 (A and B) shows the ability of one of the antisera to recognise specifically by immunoblot the recombinant polypeptide encoded by cpRL3. The antiserum recognises the recombinant polypeptide in a total lysate of bacteria in which the expression of the cpRL3 insert is induced with IPTG (Fig. 1A, lane 3). The antiserum also recognize the polypeptide after purification by the nickel chelate chromatography. The specificity of the reaction is demonstrated by the lack of reactivity against *E. coli* proteins, as well as against an unreiated recombinant protein, TRAP, expressed from the control plasmid pDS56/RBSII-6xHis (TRAP), also snaring the six histidine amino-terminal tail. Antibodies developed against the expression product of insert cpRL3 (mouse antiserum and monoclonal antibody 2B11) detect, in the lysate of *Cryptosporidium* oocysts, a protein of the apparent molecular weight of 190,000 Dalton, (Figure 2 A and B). These results indicates that the expression product of the insert cpRL3 is part of a *Clyptosporidium* protein expressed in the parasite oocysts.

### Example 6 Immunofluorescence microscopy

Purified parasite oocysts are air dried on a coverslip and fixed in cold acetone for 5 min. Non-specific binding is prevented by pre-incubation of the samples in PBS containing 1% bovine albumin. Primary antibodies (culture supernatant) are allowed to react for 40 min at room temperature, and the secondary fluorescinated antibody (Becton Dickinson goat anti-mouse) is allowed to react for 20 min. Observation of the samples is carried out with confocal microscopic apparatus (Bio-Rad Laboratories).

The protein is localised by immunofluorescence using the monoclonal antibody 2B11 on a preparation of oocysts fixed with acetone. The monoclonal 2B11 specifically binds to a protein of oocyst wall of *Cryptosporidium.* The result of the immunolocalization is confirmed by confocal immunofluorescence analysis which shows that the reactivity is detected on the surface of the oocyst wall. On the basis of these observations, the Cryptosporidium protein encoded by the insert cpRL3 is named as Cryptosporidium oocyst wall protein, COWP.

By immunofluorescence, monoclonal antibodies can identify as few as 100 oocysts/ml of stoois. The detection limit of this method is determined resuspending known numbers of oocysts in samples of non infected stools.

### Example 7 ELISA assays

Monoclonal antibodies produced against the product of the insert cpRL3 are able to recognise COWP in solution when used in ELISA assays based on antigen capture with two antibodies. Therefore specific antibodies directed against this protein or fragments thereof having an antigenic activity, as the product of cpRL3, can be employed to develop diagnostic assay to reveal *Cryptosporidium* infection.

### Example 8 PCR

For all PCR experiments, the cpRL3 sequence is amplified in a standard 50 µl PCR reaction mixture (Saiki, R K., et al. 1988. Science 239:487-491; Scharf, S. J. et al. 1986. Science 233:1076-1078) for 35 cycles at 94,5°C (1 min), 58°C (30 sec); 72°C(1 min), with a Lab Line thermal cycler. The final concentration of MgCl₂ is 2 mM. Primers used are: non coding strand, nt. 1828-1852 of complementary strand of SEQ ID No. 1.

For amplification of the cpRL3 sequence from Cryptosporidium oocysts, samples are incubated for 5 min under reducing conditions and boiled for 10 min thereafter. TaqI polymerase is purchased from Perkin-Elmer Co.

The ability of two oligonucleotide combinations to amplify by PCR the sequence of cpRL3 is shown in Figure 3A. Both of oligonucleotide combinations Cry3/Cry6 and Cry5/Cry6 are able to amplify DNA segments of the expected molecular weight from the plasmid pDS56/RESII-E-6xHis (cpRL3) and from DNA of *Cryptosporidium.* The amplification reaction using oligonucleotides Cry3/Cry6 is highly specific, (figure 3B). Oligonucleotides in fact do not amplify any DNA segment when DNA of other protozoa *(Giardia lamblia, Plasmodium falciparum o Sarcocystis suis hominis*) is used as template. In PCR experiments the oligonucleotide combination Cry3/Cry6 is able to amplify a specific DNA segment from as few as 40 oocysts of *Cryptosnoridium,* (Figure 3C). These data indicate that by using combination of oligonucleotides corresponding different regions of cpRL3 sequence, PCR can be employed to detect the presence of *Cryptosporidium.*

## Claims

1. A polypeptide of the oocyst wall of *Cryptosporidium* having a molecular weight of 190.000 D as determined by SDS polyacrilamide gel electrophoresis and comprising a contiguous sequence encoded by a nucleotide sequence of SEQ ID No.1 or parts or variants thereof reacting with antibodies developed against the expression product of the nucleotide sequence of SEQ ID No.1.

2. A polypeptide according to claim 1 consisting of a contiguous sequence encoded by the nucleotide sequence of SEQ ID No.1 or by parts or variants thereof reacting with antibodies developed against the expression product of the nucleotide sequence of SEQ ID No.1.

3. A polypeptide according to claim 2 wherein said contiguous sequence is comprised in the aminoacid sequence of SEQ ID No.2.

4. A diagnostic kit for the detection of *Cryptosporidium* in biological and environmental samples comprising, as specific ligand, the polypeptide according to any of previous claims.

5. Use of a polypeptide according to any of claims from 1 to 3 for raising antibodies able to detect *Cryptosporidium* infection in biological and environmental samples.

6. An antibody obtained using as immunogen a polypeptide according to any of claims from 1 to 3.

7. Use of the antibody according to claim 6 for the specific detection of *Cryptosporidium* or of *Cryptosposridium* oocyst wall protein.

8. A diagnostic kit for the detection of *Cryptosporidium* in biological and environmental samples comprising, as specific ligand, an antibody able to react with at least one polypeptide according to any of claims from 1 to 3.

9. An oligonucleotide having a sequence comprised in the nucleotide sequence of SEQ ID No.1, or in the complementary strand of SEQ ID No.1 able to hybridize selectively to nucleotide sequences of *Cryptosporidium.*

10. A diagnostic kit for the detection of *Cryptosporidium* in biological and environmental samples comprising, as specific ligand, the oligonucleotide according to claim 9.

11. A PCR kit for the amplification of *Cryptosporidium* DNA comprising, as specific primer, at least one oligonucleotide according to claim 9.

12. A PCR kit according to Claim 11 comprising, as specific primer, two oligonucleotides according to claim 9.

13. A PCR kit according to claim 12 wherein the two oligonucleotides have the sequence from nt. 722 to nt. 742 of SEQ ID No. 1 and from nt. 1828 to nt. 1852 of the complementary strand of SEQ ID No. 1, respectively.

## Patentansprüche

1. Polypeptid der Oocystwand von Cryptosporidium mit einem Molekulargewicht von 190.000 D, wie durch SDS Polyakrilamudgel-Elektrophorese bestimmt und enthaltend eine benachbarte, durch eine Nucleotid Sequenz von SEQ ID Nr. 1 oder deren Teile bzw. Varianten kodifizierte Sequenz, die mit Antikörpern reagiert, welche gegen des Expressionsprodukt der Nucleotid-Sequenz von SEQ ID Nr. 1 entwickelt sind.

2. Polypeptid nach Anspruch 1 bestehend aus einer benachbarten, durch eine Nucleotid-Sequenz von SEQ ID Nr. 1 oder deren Teile bzw. Varianten kodifizierten Sequenz, die mit Antikörpern reagiert, welche gegen das Expressionsprodukt der Nucleotid-Sequenz von SEQ ID Nr. 1 entwickelt sind.

3. Polypeptid nach Anspruch 1, worin die vorgenannte benachbarte Sequenz in der Aminosäure-Sequenz von SEQ ID Nr.2 enthalten ist.

4. Diagnostik-Kit zur Entdeckung von Cryptosporidium in biologishen und Umwelt-Mustern, enthatend, als spezifischer Binder, das Polypeptid nach je einem der vorhergehenden Ansprüche.

5. Anwendung eines Polypeptids nach je einem der vorhergehenden Ansprüche 1 bis 3 zur Vermehrung von Antikörpern, die eine auf Cryptosporidium zurückzuführende Infektion festzustellen imstande sind.

6. Antikörper erhalten bei Anwendung, als Immunogen, eines Polypeptids nach je einem der Ansprüche 1 bis 3.

7. Anwendung des Antikörpers nach Anspruch 6 zur spezifischen Feststellung von Cryptosporidium oder Oocystwandprotein von Cryptosporidium.

8. Diagnostik-Kit zur Feststellung von Cryptosporidium in biologishen oder Umwelt-Mustern, enthaltend, als spezifischer Binder, einen Antikörper, der mit mindestens einem Polypeptid nach je einem der Ansprüche 1 bis 3 zu reagieren imstande ist.

9. Oligonucleotid mit einer in der Nucleotid-Sequenz von SEQ ID Nr. 1 oder in einer Komplentarkette von SEQ ID Nr. 1 enthaltenen Sequenz, die an die Nucleotid-Sequenz von Cryptosporidium selektiv zu hybridisieren imstande ist.

10. Diagnostik-Kit zur Feststellung von Cryptosporidium in biologishen oder Umwelt-Mustern, enthaltend, als spezifischer Binder, das Oligonucleotid nach Anspruch 9.

11. PCR-Kit zur Verstärkung von Cryptosporidium DNA, enthaltend, als spezifisches Anregnungsmittel, mindestens ein Oligonucleotid nach Anspruch 1.

12. PCR-Kit nach Anspruch 11, enthaltend, als spezifischer Anregnungsmittel, zwei Oligonucleotide nach Anspruch 9.

13. PCR-Kit nach Anspruch 12, worin die zwei Oligonucleotide die Sequenz Nt. 72 bis 742 von SEQ ID Nr. 1 bzw. Nt. 1828 bis Nt. 1852 der Komplementer-Kette von SEQ ID Nr. 1 haben.

## Revendications

1. Polypeptide de paroi oocystique de Cryptosporidium ayant un poids moléculaire de 190.000 D, comme determiné par l'éléctrophorèse de gel de polyacrilamide SDS et comprenant une séquence contigue codifiée par une séquence nucléotidique de SEQ ID No. 1 ou ses portions ou variantes capable de réagir avec des anticorps développés contre le produit d'expression de la séquence nucléotidique de SEQ ID No. 1.

2. Polypeptide selon la revendication 1, consistant d'une séquence contigue codifiée par la séquence nucléotidique de SEQ ID No. 1 ou ses portions ou variantes capable de réagir avec des anticorps développés contre le produit d'expression de la séquence nucléotidique de SEQ ID No. 1.

3. Polypeptide selon la revendication 2, dans lequel ladite séquence contigue est contenue dans la séquence aminoacide de SEQ ID No. 2.

4. Matériel diagnostique pour la détection de Cryptosporidium dans des échantillons biologique et du milieu, comprenant, comme liant spécifique, le polypeptide selon l'une quelconque des revendications précédentes.

5. Usage d'un polypeptide selon l'une quelconque des revendicantions 1 à 3 pour augmenter les anticorps capable de détecter un infection de Cryptosporidium dans des échantillons biologiques et du milieu.

6. Anticorps obtenu par l'usage, comme immunogène, d'un polypeptide selon l'une quelconque des revendications 1 à 3.

7. Usage de l'anticorps selon la revendication 6 pour la détection spécifique de Cryptosporidium ou de protéine de paroi oocystique de Cryptosporidium.

8. Matériel diagnostique pour la détection de Cryptosporidium dans des échantillons biologiques et du milieu, comprenant, comme liant spécifique, un anticorps capable de réagir avec au moins un polypeptide selon l'une quelconque des revendications 1 à 3.

9. Oligonucléotide ayant une séquence contenue dans la séquence nucléotidique de SEQ ID No. 1, ou dans la chaîn complémentaire de SEQ ID No. 1 capable de hybrider selectivement à les séquences nucléotidiques de Cryptosporidium.

10. Matériel diagnostique pour la détection de Cryptosporidium dans les champions biologiques et du milieu, comprenant, comme liant spécifique, l'oligonucléotide selon la revendication 9.

11. Matériel de PCR pour l'amplification de Cryptosporidium DNA comprenant, comme stimulant spécifique, au moins un oligonucléotide selon la revendication 9.

12. Matériel de PCR selon la revendication 11, comprenant, comme stimulant spécifique, au moins deux oligonucléotides selon la revendication 9.

13. Matériel de PCR selon la revendication 12, dans lequel les deux oligoucléotides ont la séquence de nt 722 à nt 742 de SEQ ID No. 1 et de nt. 1828 à nt. 1852 de la chaîn complémentaire de SEQ ID No. 1, respectivement.
